# EUROPEAN PATENT SPECIFICATION

(11) **EP 1 516 922 B1**
(45) Date of publication and mention of the grant of the patent: **22.04.2009**
(21) Application number: 04255731.4
(22) Date of filing: 21.09.2004
(51) Int. Cl.: C12N 5/00, A61L 27/00

(54) **Apparatus for use in the regeneration of structured human tissue**
Anordnung zur Verwendung in der Regeneration des strukturierten menschlichen Gewebes
Appareil pour l'usage dans la régénération du tissu humain structuré

(30) Priority: 22.09.2003 GB 0322145
(43) Date of publication of application: 23.03.2005
(73) Proprietor: Steinwachs, Matthias, 8704 Herrliberg (CH)
(72) Inventor: Steinwachs, Matthias, 8704 Herrliberg (CH)
(74) Representative: Köpf, Alfred

(56) References cited:
- WO-A-00/32251
- US-A- 5 736 372
- US-A1- 2002 173 806
- US-A1- 2002 182 241

## Description

This application relates to apparatus for use in the regeneration of structured human tissue and is particularly, although not exclusively, applicable for meniscus, disc, tendon and ligament applications.

US 2002/182241 A1 discloses a tissue scaffold device consisting of two joined or fastened layers of biocompatible degradable polymer material. The layers are produced with a micro-fabricated mold. Between the two layers an array of channels is arranged, mimicking a vascular vessel network. Endothelial cells are added to the vessels, which subsequently line the channels. A three-dimensional scaffold is achieved by rolling or stacking the device.

According to the present invention apparatus for use in the regeneration of structured human tissue comprises a pair of opposed surfaces of bio material or a synthetic polymer material, each surface carrying an active bio layer which can interact with stem cells from bone marrow, and which can be moved from an open position where the opposed surfaces are spaced apart to a closed position in which they are closer together to form a multi layer or sandwich construction.

The bio material or synthetic polymer material is preferably flexible and in one preferred construction the opposed surfaces are provided on a folded sheet. In another convenient construction the opposed surfaces can be provided on the inner surfaces of a tube.

Means can be provided for holding the surfaces in the closed position, for example by integrated sutures, filaments or bands.

Means can also be provided for holding the surfaces in position on a bone or other human organ for example a meniscus, disc, tendon or ligament.

In one preferred embodiment the bio material is of Collagen I type and the active bio layer is Hyaluronic acid, or the bio material is a combination of a natural and synthetic polymer with an active bio layer.

The synthetic polymer material can be a polyvinyl alcohol, acetate hydrogel, polycaprolactone, polyurethane, alpha and beta hydroxy acids or esters and combinations thereof, or other suitable porous or biogradable polymers that can be fabricated in the form of a scaffold or matrix to support the active bio layer.

The construction of the apparatus is such that it enables the active bio layer to be impregnated with stem cells from bone marrow taken from the patient and prior to closing the surfaces together. The advantage is that even in a deep bio layer a homogeneous distribution of the stem cells in the form of bone marrow blood can be achieved. With existing techniques this is not possible. Additionally it enables an impregnation of any other cells, growth factors, cytokines, coating materials, and any other bioactive materials.

The invention can be performed in various ways and some embodiments will now be described by way of example and with reference to the accompanying drawings in which :
Figure 1 is a pictorial oblique view of the apparatus according to the invention ready for use;
Figure 2 shows how filtrated human bone marrow (blood) can be applied to the opened surfaces;
Figure 3 shows the surfaces according to the apparatus in the closed position and ready for use;
Figure 4 is a pictorial representation of a tube embodying the invention;
Figure 5 shows the tube in a closed position;
Figure 6 shows how the opposed surfaces of bio material or synthetic polymer material can be provided as two separate elements; and
Figure 7 is a pictorial oblique view showing the overall shape of another construction according to the invention.

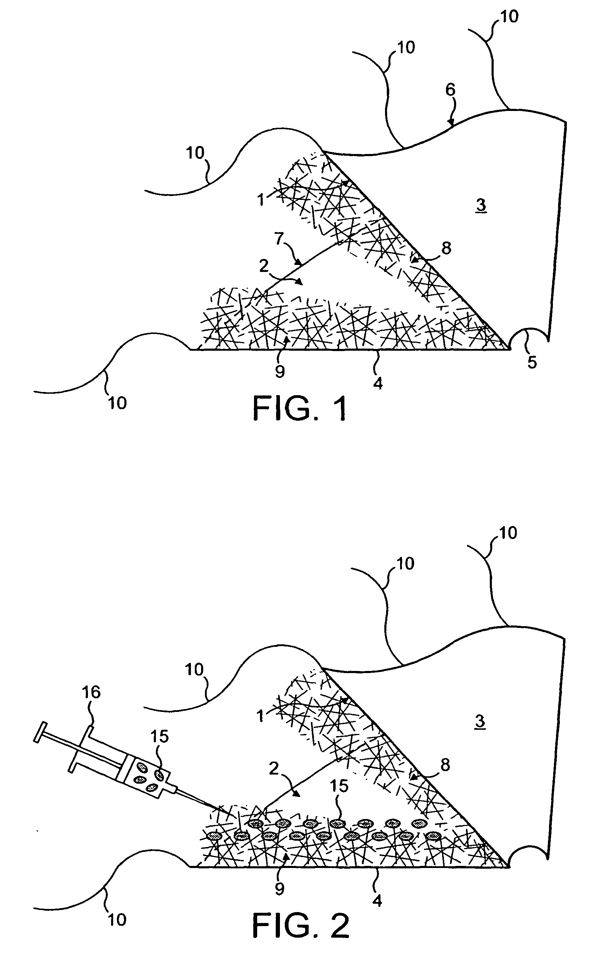

As shown in Figures 1 to 3 the apparatus for use in the regeneration of structured human tissue according to the present invention comprises a pair of opposed surfaces 1 and 2 which are formed on a sheet or film of bio material, for example Collagen I or synthetic polymers and other resorbable materials. The sheet has a double triangular or wedge configuration, surface 1 being on a first generally triangular shaped sheet portion 3 and surface 2 on a second triangular sheet portion 4. The adjoining ends of the two generally triangular portions 3 and 4 are indicated by joining portion 5 and the opposing sides are indicated by reference numerals 6 and 7. The surfaces 1 and 2 each carry an active bio layer indicated by reference numerals 8 and 9. In order to make the drawing more clear only the edges of the bio layer are shown on each surface. The bio material or synthetic polymer material is flexible.

Ties in the form of stitches or bands 10 are provided so that the surfaces can be held together in a closed position.

Figure 2 shows how the stem cells, cytokines, growths factors, other cells, harvested from bone marrow, indicated by reference numeral 15, in a syringe 16, can be applied to one of the active bio layers 9. The stem cells are provided by filtrated human bone marrow in the form of bone marrow blood taken from the patient. This can be done by creating a drill hole into an appropriate bone, aspirating the bone marrow blood and filtrating it.

With the apparatus according to the invention even in a deep bio layer a homogeneous distribution of the blood solution can be achieved.

The blood solution will include cells of (hMSC fibrocytes, chondrocytes) and proteins (cytokines, growth factors).

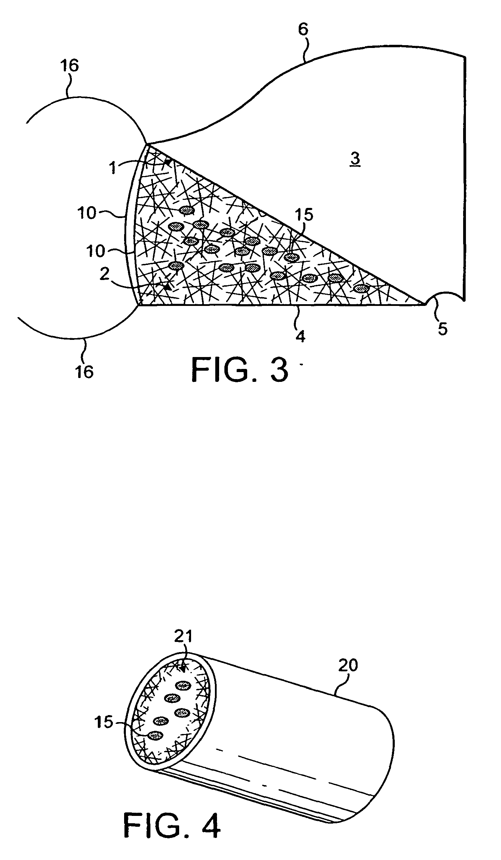

Figure 3 shows how the surfaces 1 and 2 can be folded together to a closed position and can be held in place by the ties 10. The ties 10 can be in the form of stitches or bands which are integrated into the material.

Additionally bands of the synovia which are prepared during surgery can be used for fixation purposes. The synovia laps can surround the construct or just cover a surface.

Additional anchoring ties 16 can be provided as shown in Figure 3 and these are used to attach the apparatus to the appropriate bone or human organ, for example a meniscus, disc, tendon or ligament.

As will be seen from the drawings a sandwich construction is achieved.

When installed the apparatus will be partially surrounded by synovia and fibrochondrocytes, hMSC's and growth factors and cytokines an other cells from the bone marrow blood or external coating together with local blood vessels will all assist in the regeneration of structured human tissue which is required.

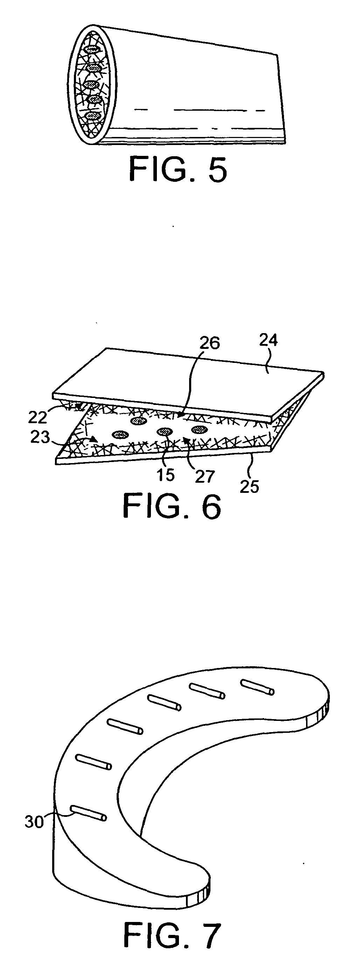

Figure 4 shows an alternative construction for providing a pair of opposed surfaces of bio material or synthetic polymer material which comprises a tube 20 of the appropriate material and the inner surface of which carries an active bio layer 21. As in the previous Figures only the edge of the bio layer is shown in the drawing. The filtrated human bone marrow 15 is applied onto the active bio layer 21 whilst the tube is in the open position shown in Figure 4. The tube can now be moved to a closed position, as shown in Figure 5, where a sandwich construction is again achieved. Ties 10 and 16 can again be provided but are not shown in the drawing.

In another alternative construction, as shown in Figure 6, the surfaces 22 and 23 are formed on two flat sheets 24 and 25 of bio material or synthetic polymer material. Active bio layers 26 and 27 are provided on the sheets. As in the previous embodiments only the edges of the layers are shown. Filtrated human bone marrow or other cells, cytokines, growth factors or coating materials can again be injected onto the bio layer 27 and the sheets closed together by ties 10 (not shown). Ties 16 can also be included to hold the surfaces in the closed position.

Figure 7 is a pictorial oblique view of the overall general shape of another construction according to the invention. The overall shape is of a crescent shaped meniscus which can be fitted in position in the manner shown in Figures 1, 2 and 3. This Figure also shows how the bio material or synthetic polymer material can carry a number of tubes, indicated by reference numeral 30. These tubes will allow, by capillary action the injection of the cells to be distributed within the material. Tubes of this kind can also be employed in any of the constructions shown in the other Figures.

## Claims

1. Apparatus for use in the regeneration of structured human tissue comprising a pair of opposed surfaces of bio material or a synthetic polymer material, each surface carrying an active bio layer which can interact with stem cells from bone marrow, and which can be moved from an open position where they are spaced apart to a closed position in which they are closer together to form a multi layer or sandwich construction.

2. Apparatus as claimed in claim 1 in which said opposed surfaces are provided on a folded sheet.

3. Apparatus as claimed in claim 1 in which said opposed surfaces are provided on the inner surfaces of a tube.

4. Apparatus as claimed in claims 1 to 3 including means for holding the surfaces in the closed position.

5. Apparatus as claimed in claims 1 to 4 including means for holding the surfaces in position on a bone, or other human organ.

6. Apparatus as claimed in claim 5 in which the human organ is a meniscus, disc, tendon or ligament.

7. Apparatus as claimed in any of the preceding claims in which the bio material is of Collagen I type and the active bio layer is Hyaluronic acid.

8. Apparatus as claimed in any of the preceding claims 1 to 6 in which the bio material is a combination of a natural and synthetic polymer with an active bio layer.

9. Apparatus as claimed in any of the preceding claims 1 to 8 in which the synthetic polymer material is a polyvinyl alcohol, acetate hydrogel, polycaprolactone, polyurethane, alpha and beta hydroxy acids or esters and combinations thereof, or other suitable porous or biogradable polymers that can be fabricated in the form of a scaffold or matrix to support the active bio layer.

## Patentansprüche

1. Vorrichtung zur Verwendung bei der Regeneration von strukturiertem, menschlichem Gewebe, umfassend ein Paar von gegenüberliegenden Oberflächen von Biomaterial oder einem synthetischen Polymermaterial, wobei jede Oberfläche eine aktive Bioschicht tragt, die mit Stammzellen aus Knochenmark wechselwirken kann, und die von einer offenen Position, bei der sie getrennt angeordnet sind, zu einer geschlossenen Position bewegt werden können, bei der sie näher zusammen angeordnet sind, um einen Mehrschicht- oder Sandwich-Aufbau zu bilden.

2. Vorrichtung gemäß Anspruch 1, wobei die gegenüberliegenden Oberflächen auf einer gefalteten Folie bereitgestellt sind.

3. Vorrichtung gemäß Anspruch 1, wobei die gegenüberliegenden Oberflächen auf der Innenoberfläche eines Rohrs bereitgestellt sind.

4. Vorrichtung gemäß Ansprüchen bis 3, umfassend Mittel zum Festhalten der Oberflächen in der geschlossenen Position.

5. Vorrichtung gemäß Ansprüchen 1 bis 4, umfassend Mittel zum Festhalten der Oberflächen in Position auf einem Knochen oder einem anderen menschlichen Organ.

6. Vorrichtung gemäß Anspruch 5, wobei das menschliche Organ ein Meniskus, ein Diskus, eine Sehne oder ein Band ist.

7. Vorrichtung gemäß einem der vorstehenden Ansprüche, wobei das Biomaterial aus Collagen Typ I besteht und die aktive Bioschicht Hyaluronsäure ist.

8. Vorrichtung gemäß einem der vorstehenden Ansprüche 1 bis 6, wobei das Biomaterial eine Kombination eines natürlichen und synthetischen Polymers mit einer aktiven Bioschicht ist.

9. Vorrichtung gemäß einem der vorstehenden Ansprüche 1 bis 8, wobei es sich bei dem synthetischen Polymermaterial um einen Polyvinylalkohol, Acetat-Hydrogel, Polycaprolacton, Polyurethan, alpha- und beta-Hydrvxysäuren oder Ester und Kombinationen davon, oder andere geeignete poröse oder biologisch abbaubare Polymere handelt, die in der Form eines Gerüsts oder einer Matrix zum Tragen der aktiven Bioschicht gefertigt werden können.

## Revendications

1. Appareil destiné à être utilisé pour la régénération d'un tissu humain structuré, comprenant une paire de surfaces opposées d'un matériel biologique ou d'un matériel polymère synthétique, chaque surface portant une couche biologique active qui peut interagir avec des cellules souches de moelle osseuse, et lesdites surfaces pouvant être déplacées d'une position ouverte dans laquelle elles sont espacées jusqu'à une position fermée dans laquelle elles sont rapprochées de manière à former une construction multicouche ou en sandwich.

2. Appareil selon la revendication 1, dans lequel lesdites surfaces opposées sont disposées sur une feuille pliée.

3. Appareil selon la revendication 1, dans lequel lesdites surfaces opposées sont disposées sur les surfaces internes d'un tube.

4. Appareil selon les revendications 1 à 3, comprenant des moyens permettant de maintenir les surfaces en position fermée.

5. Appareil selon les revendications 1 à 4, comprenant de moyens permettant de maintenir les surfaces en place sur un os ou un autre organe humain.

6. Appareil selon la revendication 5, dans lequel l'organe humain est un ménisque, un disque, un tendon ou un ligament.

7. Appareil selon l'une quelconque des revendications précédentes, dans lequel le matériel biologique est de type collagène 1 et la couche biologique active est l'acide hyaluronique.

8. Appareil selon l'une quelconque des revendications précédentes 1 à 6, dans lequel le matériel biologique est une association d'un polymère naturel et synthétique avec une couche biologique active.

9. Appareil selon l'une quelconque des revendications précédentes 1 à 8, dans lequel le matériel polymère synthétique est un alcool polyvinylique, un hydrogel d'acétate, une polycaprolactone, du polyuréthane, des alpha- et bêta-hydroxyacides ou esters et des combinaisons de ceux-ci, ou d'autres polymères poreux ou biodégradables appropriés qui peuvent être fabriqués sous forme d'un échafaudage ou d'une matrice pour soutenir la couche biologique active.
